(19) Europäisches Patentamt
European Patent Office
Office européen des brevets

(11) Veröffentlichungsnummer: **0 022 213**
**A1**

(12) **EUROPÄISCHE PATENTANMELDUNG**

(21) Anmeldenummer: **80103571.8**

(22) Anmeldetag: **24.06.80**

(51) Int. Cl.³: **A 61 K 31/425**
//C07D277/68

(30) Priorität: **06.07.79 DE 2927352**

(43) Veröffentlichungstag der Anmeldung:
**14.01.81 Patentblatt 81/2**

(84) Benannte Vertragsstaaten:
**BE CH DE FR GB IT LI LU NL SE**

(71) Anmelder: **Dr. Karl Thomae GmbH**
**Postfach 720**
**D-7950 Biberach (Riss)(DE)**

(72) Erfinder: **Trummlitz, Günter, Dr. Dipl.-Chem.**
**Buchenweg 27**
**D-7951 Warthausen(DE)**

(72) Erfinder: **Engel, Wolfhard, Dr. Dipl.-Chem.**
**Mozartstrasse 13**
**D-7950 Biberach 1(DE)**

(72) Erfinder: **Schmidt, Günther, Dr. Dipl.-Chem.**
**Johann-Sebastian-Bach-Strasse 27**
**D-7950 Biberach 1(DE)**

(72) Erfinder: **Eberlein, Wolfgang, Dr. Dipl.-Chem.**
**Obere Au 6**
**D-7950 Biberach 1(DE)**

(72) Erfinder: **Seeger, Ernst, Dr. Dipl.-Chem.**
**Alpenstrasse 39**
**D-7950 Biberach 1(DE)**

(72) Erfinder: **Engelhardt, Günther, Dr.**
**Unterer Bühl 18**
**D-7950 Biberach 1(DE)**

(72) Erfinder: **Zimmermann, Rainer, Dr. Dipl.-Biochem.**
**Laurenbühlstrasse 17**
**D-7951 Mittelbiberach(DE)**

(54) **Benzothiazol-2(3H)-one enthaltende Arzneimittel.**

(57) Es werden Arzneimittel beschrieben, die als Wirksubstanzen Benzothiazol-2(3H)-one der allgemeinen Formel I

R–[Benzothiazol-2(3H)-on: S, =O, N–H] ,(I)

enthalten, worin R ein Wasserstoffatom, die Methyl-, Methoxy- oder Äthoxygruppe bedeutet. Die Arzneimittel sind Analgetika und wirken zusätzlich antipyretisch ohne eine nennenswerte methämoglobinbildende Wirkung zu besitzen. Die Einzeldosierung beträgt im allgemeinen 20 bis 600 mg Wirksubstanz, die Tagesdosis liegt zwischen 180 und 900 mg.

EP 0 022 213 A1

Case 5/768
Dr.Bu/pf./st
Auslandstext

DR. KARL THOMAE GMBH, BIBERACH AN DER RISS

# Benzothiazol-2(3H)-one enthaltende Arzneimittel

Die Erfindung betrifft Benzothiazol-2(3H)-one der allgemeinen Formel

,(I)

enthaltende Arzneimittel. In der allgemeinen Formel I bedeutet R ein Wasserstoffatom, die Methyl-, Methoxy- oder Ethoxygruppe.

R.F. Hunter und E.R. Parken beschrieben in einer ausschließlich chemischen Arbeit die Verbindungen 6-Methyl- und 6-Ethoxybenzothiazol-2(3H)-on (J. chem. Soc. 1935, Seite 1755), desweiteren das Benzothiazol-2(3H)-on selbst (J. Chem. Soc. 1930, Seite 125). Für die letztere Verbindung ist eine antimikrobielle und fungistatische Wirkung in der Literatur erwähnt (W. Nimmich, Pharmazie 19 [4], 281 [1964]).

Durch die Publikation Friedländer 22/1, Seite 324 (1939) (s. auch Chem. Abstr. 29, 6250 /1935/) wurde das 6-Methoxy-benzothiazol-2(3H)-on erwähnt; über eine pharmakologische Wirkung ist aber nichts ausgesagt worden. Im Schrifttum werden Benzothiazole analog der allgemeinen Formel I öfters als Zwischenprodukte für die Herstellung von Farbstoffen und Pharmazeutika genannt.

Umso überraschender war deshalb das Auffinden einer sehr guten analgetischen Wirkung neben einer ausgeprägten antipyretischen Wirkung bei einer guten Verträglichkeit bei den Verbindungen der allgemeinen Formel I. Es kommt hinzu, daß die Verbindungen der allgemeinen Formel I im Gegensatz zu ähnlich wirkenden Analgetika, z.B. Phenacetin, eine wesentlich abgeschwächte oder keine Methämoglobin-bildende Wirkung zeigen.

Die Verbindungen der allgemeinen Formel I lassen sich wie folgt herstellen:

Ein 2-Amino-thiophenol der allgemeinen Formel

R–C₆H₃(SH)(NH₂) ,(II)

in der R wie oben definiert ist, wird mit N, N'-Carbonyldiimidazol in einem wasserfreien Lösungsmittel wie Benzol, Toluol oder anderen Kohlenwasserstoffen oder wie Tetrahydrofuran, Dioxan oder ähnlichen cyclischen Äthern bei Temperaturen bis zum Siedepunkt des Reaktionsgemisches umgesetzt. Im allgemeinen genügt es, zur Aufarbeitung das Lösungsmittel im Vakuum zu entfernen und das Reaktionsprodukt beispielsweise aus 1,2-Dichloräthan, Ethanol oder Methanol umzukristallisieren.

Die als Ausgangsmaterialien verwendeten 2-Amino-thiophenole sind literaturbekannt oder sie lassen sich nach literaturbekannten Methoden herstellen. So erhält man z.B. das 2-Amino-thiophenol (Siedepunkt: 80-81°C / 3mbar, Schmelzpunkt: 92°C) durch Erhitzen von 2-Mercaptobenzothiazol mit Natronlauge unter Druck (G.G. Skvortsova, Z.V. Stepanova und S.M. Tyrina, Zh. Organ. Khim. 2 (9), 1656 /1966/), das 2-Amino-5-methyl-thiophenol (Schmelzpunkt: 90°C), das 2-Amino-5-methoxy-thiophenol (Schmelzpunkt: 104°C) und das 2-Amino-5-ethoxy-thiophenol (Schmelzpunkt: 104°C) durch Hydrolyse der entsprechenden 2-Amino-benzothiazole mit siedender wässeriger Kalilauge (siehe R.L. Mital und S.K. Jain, J. Chem. Soc. (C) 1969, 2148 bzw. S.K. Jain und R.L. Mital, Z. Naturforsch. 32 B, 821 /1977/).

Wie bereits eingangs erwähnt, besitzen die Verbindungen der allgemeinen Formel I eine sehr gute analgetische und antipyretische Wirkung bei fehlender oder nur geringer Methämoglobin-bildender Wirkung.

Die Verbindungen

6-Ethoxy-benzothiazol-2(3H)-on, = A

6-Methoxy-benzothiazol-2(3H)-on und = B

6-Methyl-benzothiazol-2(3H)-on = C

wurden im Vergleich mit Phenacetin = D auf ihre analgetische Wirkung an der Maus und Ratte, ihre antipyretische Wirkung an der Ratte, ihre Methämoglobin-bildende Wirkung an der Katze sowie ihre akute Toxizität an der Maus geprüft.

Wirkung gegen den Wärmeschmerz der Maus

Die Prüfung erfolgte mit Hilfe einer Modifikation der Versuchsanordnung nach CHEN und BECKMAN (Science 113, 631 (1951)) an männlichen Chbb: NMRI (SPF)-Mäusen mit einem mittleren Gewicht

von 20 g. Die "heiße Platte" bestand aus Aluminium und hatte eine Oberflächentemperatur von 52°C.

Die Prüfsubstanzen wurden als Verreibung in 1%iger Methylzellulose (0,1 ml/10 g Maus) per Schlundsonde verabfolgt.

Vor der Behandlung mit Prüfsubstanzen wurden die Tiere im Abstand von 30 min zweimal auf die heiße Platte gesetzt. Es wurde ihre individuelle Reaktionszeit ermittelt. Nach Substanzgabe wurde in Abständen von 30 min die Reaktionszeit erneut gemessen.

Aus der mit den verschiedenen Dosen der Prüfsubstanzen erzielten gemittelten maximalen Verlängerung der Reaktionszeit wurde nach linearer Regressionsanalyse nach LINDER (Statistische Methoden 4. Aufl., pp. 148-162, Birkhäuser, Basel 1964) eine $ED_{100}$ mit den Vertrauensgrenzen nach FIELLER (Quart. Journal Pharmacol. 17, 117-123 (1944)) als die Dosis berechnet, die zu einer 100%igen Verlängerung der Reaktionszeit führte.

Wirkung gegen den Entzündungsschmerz der Rattenhinterpfote

Die Prüfung erfolgte mit der Methode nach RANDALL-SELITTO (Arch. int. Pharmacodyn. 111, 409 (1957)) an männlichen Chbb:THOM-Ratten mit einem Gewicht zwischen 100 und 130 g. Die Prüfsubstanzen wurden 135 min nach Auslösung des Hefeödems als Verreibung in 1%iger Methylzellulose per Schlundsonde beigebracht. Weitere 45 min danach wurde bei den mit Prüfsubstanz behandelten und den nur mit dem Vehikel behandelten Kontrolltieren die Schmerzschwelle bestimmt und nach linearer Regressionsanalyse nach LINDER (s.o.) eine $ED_{50}$ mit den Vertrauensgrenzen nach FIELLER (s.o.) als die Dosis berechnet, die eine Anhebung der Schmerzschwelle um 50 % bewirkte.

Antipyretische Wirkung

Die Prüfung der temperatursenkenden Wirkung erfolgte durch die Kontrolle des Verlaufes der rektal gemessenen Körpertemperatur von teilweise immobilisierten Chbb:THOM-Ratten mit einem Gewicht zwischen 125 und 150 g. Die Prüfsubstanzen wurden als Verreibung in 1%iger Methylzellulose in einem Volumen von 1,0 ml/100 g Tier per Schlundsonde appliziert.

Aus den nach Gabe der verschiedenen Dosen der Prüfsubstanz gewonnenen Werten für die mittlere maximale Temperatursenkung wurde nach linearer Regressionsanalyse nach LINDER (s.o.) eine $ED_{-1,5}$oC als die Dosis berechnet, die eine Senkung der Körpertemperatur um 1,5°C bewirkte.

Bestimmung des Methämoglobinspiegels an der Katze

Katzen beider Geschlechter in einem mittleren Gewicht von 2,5 kg erhielten die Prüfsubstanzen als Verreibung in 1%iger Methylzellulose per Schlundsonde beigebracht (2 ml/kg; die Schlundsonde wurde mit Aqua bidest. in situ leergespült).

Blutproben wurden zu verschiedenen Zeiten nach der einmaligen Applikation des Wirkstoffes per Herzpunktion gewonnen. Die photometrische Registrierung der Hämoglobinspektren sowie die Bestimmung des Methämoglobingehaltes im Vergleich zur Kontrolle erfolgte nach LEMBERG und LEGGE (Hematin compounds and bile pigments, Interscience Publishers, New York, (1949)).

Akute Toxizität an der Maus

Die Bestimmung der akuten Toxizität erfolgt an Chbb:NMRI (SPF)-Mäusen beider Geschlechter in einem mittleren Gewicht von 20 g. Die Prüfsubstanzen wurden als Verreibung in 1%iger Methylzellulose (0,5 ml/10 g Tier) per Schlundsonde verabfolgt. Die Berechnung der $LD_{50}$ erfolgte nach LITCHFIELD und WILCOXON (J. Pharmacol. exp. Ther. 96, 99 (1949)) aus dem Prozentsatz der Tiere, die nach den verschiedenen Dosen innerhalb von 14 Tagen verstarben.

Die Verbindungen A bis C erweisen sich gegenüber dem Wärmeschmerz der Maus nach oraler Gabe als etwa doppelt so wirksam wie das bekannte Phenacetin = Verbindung D (siehe Tabelle 1). Auch an der Ratte ist die analgetische Wirkung der Verbindungen A bis C 2 mal bis 5 mal stärker als die des Phenacetins (siehe Tabelle 2).

Die Verbindungen A bis C zeigen wie das Phenacetin neben der analgetischen eine antipyretische Wirkungsqualität (siehe Tabelle 3). Während die akute Toxizität der Verbindungen A bis C weitgehend der des Phenacetins entspricht (siehe Tabelle 5), bleiben die Verbindungen A bis C im Gegensatz zu Phenacetin entweder gänzlich frei von einer Methämoglobinbildenden Wirkung oder aber diese Nebenwirkung ist bei ihnen erheblich schwächer ausgeprägt als bei Phenacetin (siehe Tabelle 4).

Tabelle 1

Wirkung gegen den Wärmeschmerz der Maus nach oraler Gabe

| Substanz | $n_1$ | $n_2$ | $ED_{100}$ | mg/kg Vertrauensgrenzen bei 95%iger Wahrscheinlichkeit |
|---|---|---|---|---|
| A | 3 | 10-20 | 154,1 | (83,4 - 208,3) |
| B | 3 | 10 | 122,0 | (58,9 - 154,4) |
| C | 3 | 10 | 143,9 | (78,0 - 197,2) |
| D | 5 | 10 | 327,7 | (208,9 - 431,8) |

$n_1$ = Anzahl der geprüften Dosen
$n_2$ = Anzahl der Tiere/Dosis

Tabelle 2

Wirkung gegen den Entzündungsschmerz an der Rattenhinterpfote in der Versuchsanordnung nach RANDALL-SELITTO 45 min nach oraler Gabe

| Substanz | $n_1$ | $n_2$ | $ED_{50}$ | mg/kg Vertrauensgrenzen bei 95%iger Wahrscheinlichkeit |
|---|---|---|---|---|
| A | 3 | 10 | 64,3 | (56,8 - 74,5) |
| B | 3 | 10 | 142,2 | (129,6 - 158,5) |
| C | 3 | 10 | 54,4 | (50,3 - 59,0) |
| D | 3 | 10 | 273,8 | (250,0 - 304,2) |

Tabelle 3

Wirkung auf die Körpertemperatur der normothermen Ratte nach oraler Gabe

| Substanz | $n_1$ | $n_2$ | $ED_{-1,5^\circ C}$ mg/kg | Vertrauensgrenzen bei 95%iger Wahrscheinlichkeit |
|---|---|---|---|---|
| A | 3 | 11-12 | 84,3 | (73,4 - 95,4) |
| B | 4 | 8-12 | 66,8 | (57,1 - 78,4) |
| C | 3 | 9 | 30,9 | (23,9 - 36,9) |
| D | 4 | 9-11 | 34,4 | (28,5 - 41,7) |

Tabelle 4

Methämoglobinspiegel der Katze nach oraler Gabe (Methämoglobin in % des Gesamthämoglobins)

| Substanz | Dosis mg/kg | 3 h n. Applikation | | | 5 h n. Applikation | | | 24 h n. Applikation | | | 48 h n. Applikation | | |
|---|---|---|---|---|---|---|---|---|---|---|---|---|---|
| | | n | $\bar{x}$ | SD | n | $\bar{x}$ | SD | n | $\bar{x}$ | SD | n | $\bar{x}$ | SD |
| | 50 | 3 | 0,3 | 0,4 | 3 | 0,5 | 0,4 | 3 | 1,1 | 1.3 | | | |
| A | 100 | 5 | 0,4 | 0,5 | 5 | 0,1 | 2,2 | 5 | 0,3 | 0,7 | | | |
| | 200 | 5 | 1,0 | 1,7 | 5 | 0,4 | 0,6 | 5 | 0,5 | 1,0 | | | |
| B | 100 | 5 | 4,6 | 4,2 | 5 | 3,5 | 5,0 | 4 | 0,0 | 0,1 | | | |
| | 200 | 5 | 8,1 | 6,6 | 5 | 11,7 | 5,9 | 5 | 0,0 | 0,2 | | | |
| C | 100 | 4 | 4,0 | 6,1 | 4 | 2,4 | 4,2 | 4 | 0,1 | 0,4 | | | |
| | 200 | 7 | 13,6 | 10,6 | 7 | 13,7 | 8,8 | 7 | 0,4 | 0,9 | | | |
| Ver- | 25 | 4 | 1,9 | 2,1 | 4 | 0,4 | 0,3 | - | | | | | |
| gleichs- | 50 | 7 | 19,6 | 9,6 | 7 | 9,8 | 6,7 | 7 | 0,5 | 0,8 | | | |
| substanz | 100 | 12 | 32,0 | 17,7 | 10 | 35,8 | 17,2 | 12 | 0,5 | 0,6 | | | |
| D | 200 | 10 | 36,0 | 17,2 | 10 | 46,6 | 16,4 | 13 | 15,4 | 22,1 | 9 | 1,1 | 1,5 |

Kontrollwert für den Methämoglobinspiegel vor Behandlung: $n = 54$, $\bar{x} = 0{,}03$ %, SD = 0,9 %

n = Anzahl der Tiere, $\bar{x}$ = Mittelwert, SD = Standardabweichung.

Tabelle 5

Akute Toxizität an der Maus nach oraler Gabe bei einer Nachbeobachtungszeit von 14 Tagen.

| Substanz | $n_1$ | $n_2$ | $LD_{50}$ | mg/kg Vertrauensgrenzen bei 95%iger Wahrscheinlichkeit |
|---|---|---|---|---|
| A | 3 | 10-20 | 1200 | (984 - 1464) |
| B | 3 | 10 | 1280 | (1130 - 1450) |
| C | 3 | 10 | 2160 | (1460 - 3200) |
| D | 3 | 10-20 | 1459 | (1231 - 1709) |

Die nachfolgenden Beispiele sollen die Erfindung näher beschreiben:

Beispiel 1

6-Ethoxy-benzothiazol-2(3H)-on

1,69 g (0,01 Mol) 2-Amino-5-ethoxy-thiophenol und 1,78 g (0,011 Mol) N,N'-Carbonyldiimidazol werden in 100 ml wasserfreiem Benzol 2 Stunden gerührt und anschließend 1 Stunde unter Rückfluß gekocht. Man filtriert das Reaktionsgemisch, dampft vom Filtrat das Lösungsmittel bei vermindertem Druck ab, kristallisiert den verbleibenden Rückstand aus 1,2-Dichlorethan um und erhält 820 mg (42 % der Theorie) 6-Ethoxy-benzothiazol-2(3H)-on.

Schmelzpunkt: 147°C.

$C_9H_9NO_2S$ (195,24)

| | C | | H | | N | | S | |
|---|---|---|---|---|---|---|---|---|
| Ber.: | C | 55,37 | H | 4,65 | N | 7,17 | S | 16,42 |
| Gef.: | | 55,30 | | 4,54 | | 7,05 | | 16,12 |

Beispiel 2

Benzothiazol-2(3H)-on

Hergestellt analog dem Beispiel 1 aus 2-Amino-thiophenol und N,N'-Carbonyldiimidazol in Tetrahydrofuran; Ausbeute: 79 % der Theorie;

Schmelzpunkt: 136 - 137°C (Methanol)

$C_7H_5NOS$ (151,19)

| | C | | H | | N | | S | |
|---|---|---|---|---|---|---|---|---|
| Ber.: | C | 55,61 | H | 3,33 | N | 9,27 | S | 21,21 |
| Gef.: | | 55,60 | | 3,48 | | 9,26 | | 21,00 |

Beispiel 3

6-Methyl-benzothiazol-2(3H)-on

Hergestellt analog Beispiel 1 aus 2-Amino-5-methyl-thiophenol und N,N'-Carbonyldiimidazol; Ausbeute: 50 % der Theorie; Schmelzpunkt: 169 - 170°C (Ethanol).

$C_8H_7NOS$ (165,22)

| | | | | | | | | |
|---|---|---|---|---|---|---|---|---|
| Ber.: | C | 58,16 | H | 4,27 | N | 8,48 | S | 19,40 |
| Gef.: | | 58,25 | | 4,20 | | 8,62 | | 19,55 |

Beispiel 4

6-Methoxy-benzothiazol-2(3H)-on

Hergestellt analog Beispiel 1 aus 2-Amino-5-methoxy-thiophenol und N,N'-Carbonyldiimidazol; Ausbeute: 47 % der Theorie; Schmelzpunkt: 163,5 - 164°C (1,2-Dichlorethan).

$C_8H_7NO_2S$ (181,22)

| | | | | | | | | |
|---|---|---|---|---|---|---|---|---|
| Ber.: | C | 53,02 | H | 3,89 | N | 7,73 | S | 17,69 |
| Gef.: | | 53,25 | | 3,90 | | 7,65 | | 17,65 |

Die Verbindungen der allgemeinen Formel I lassen sich in die üblichen pharmazeutischen Zubereitungsformen, wie Tabletten, Dragées, Suppositorien, Kapseln oder Säfte einarbeiten. Die Einzeldosierung beträgt hierbei 20 bis 600 mg für Erwachsene, vorzugsweise 50 bis 300 mg, dies entspricht einer Tagesdosierung von 60 bis 1800 mg, vorzugsweise von 180 bis 900 mg.

Die nachfolgenden Beispiele sollen die Herstellung einiger pharmazeutischer Zubereitungen verdeutlichen:

Beispiel I

Tabletten mit 50 mg 6-Äthoxy-benzothiazol-2(3H)-on

Zusammensetzung:
1 Tablette enthält:

| | |
|---|---|
| Wirkstoff | 50,0 mg |
| Milchzucker | 128,0 mg |
| Kartoffelstärke | 40,0 mg |
| Magnesiumstearat | 2,0 mg |
| | 220,0 mg |

Herstellungsverfahren:

Aus Kartoffelstärke wird durch Erwärmen ein 10%iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45°C getrocknet, nochmals durch obiges Sieb gerieben, mit Magnesiumstearat vermischt und zu Tabletten verpreßt.

Tablettengewicht: 220 mg
Stempel: 9 mm

Beispiel II

Dragées mit 50 mg 6-Äthoxy-benzothiazol-2(3H)-on

Die nach Beispiel I hergestellten Tabletten werden nach bekannten Verfahren mit einer Hülle überzogen, die im wesentlichen aus Zucker und Talkum besteht. Die fertigen Dragées werden mit Hilfe von Bienenwachs poliert.

Dragéegewicht: 300 mg

Beispiel III

Kapseln mit 60 mg 6-Äthoxy-benzothiazol-2(3H)-on

Zusammensetzung:
1 Kapsel enthält:

| | |
|---|---|
| Wirkstoff | 60,0 mg |

Herstellungsverfahren:

Der Wirkstoff wird mikronisiert und in Kapseln abgefüllt, letztere werden anschließend verschlossen.

Beispiel IV

Suppositorien mit 60 mg 6-Äthoxy-benzothiazol-2(3H)-on

Zusammensetzung:
1 Zäpfchen enthält:

| | |
|---|---|
| Wirkstoff | 60,0 mg |
| Zäpfchenmasse (z.B. Witepsol W 45®) | 1 640,0 mg |
| | 1 700,0 mg |

Herstellungsverfahren:

Die feinpulverisierte Wirksubstanz wird in der geschmolzenen und auf 40°C abgekühlten Zäpfchenmasse suspendiert. Man gießt die Masse bei 37°C in leicht vorgekühlte Zäpfchenformen aus.

Zäpfchengewicht: 1,7 g

Beispiel V

Tabletten zu 200 mg 6-Äthoxy-benzothiazol-2(3H)-on

1 Tablette enthält:

| | |
|---|---|
| Acetylsalicylsäure | 250,0 mg |
| Wirksubstanz | 200,0 mg |
| Coffein | 50,0 mg |
| Maisstärke, direkttablettierbar | 50,0 mg |
| Milchzucker, direkttablettierbar | 90,0 mg |
| Stearin-Talkum (2+8-Verreibung) | 10,0 mg |
| | 650,0 mg |

Herstellung:

Die Substanzen werden gleichmäßig gemischt und zu Tabletten verpreßt.

Tablettengewicht: 650 mg

Durchmesser: 13 mm, biplan, beidseitige Facette und einseitige Teilkerbe.

Beispiel VI

Tabletten zu 200 mg 6-Äthoxy-benzothiazol-2(3H)-on

1 Tablette enthält:

| | |
|---|---|
| Wirksubstanz | 200,0 mg |
| Milchzucker | 120,0 mg |
| Maisstärke | 70,0 mg |
| Polyvinylpyrrolidon | 8,0 mg |
| Magnesiumstearat | 2,0 mg |
| | 400,0 mg |

Herstellung:

Die Wirksubstanz, der Milchzucker und die Maisstärke werden mit einer wäßrigen Lösung von Polyvinylpyrrolidon gleichmäßig befeuchtet, durch ein Sieb mit 2 mm-Maschenweite gesiebt und im Umlufttrockenschrank bei 50°C getrocknet. Nach erneuter Siebung durch 1,5 mm-Maschenweite wird Magnesiumstearat zugemischt und die Mischung zu Tabletten verpreßt.

| | |
|---|---|
| Tablettengewicht: | 400 mg |
| Durchmesser: | 11 mm, rund, biplan, beidseitige Facette und einseitige Teilkerbe. |

Beispiel VII

Tabletten zu 200 mg 6-Äthoxy-benzothiazol-2(3)-on

1 Tablette enthält:

| | |
|---|---|
| Wirksubstanz | 200,0 mg |
| Codeinphosphat | 15,0 mg |
| Milchzucker | 110,0 mg |
| Maisstärke | 65,0 mg |
| Polyvinylpyrrolidon | 8,0 mg |
| Magnesiumstearat | 2,0 mg |
| | 400,0 mg |

Herstellung:

Die Wirksubstanz, das Codeinphosphat, der Milchzucker und die Maisstärke werden mit einer wäßrigen Lösung von Polyvinylpyrrolidon befeuchtet, durch ein Sieb von 2,0 mm-Maschenweite gesiebt, im Umlufttrockenschrank bei 50°C getrocknet, erneut gesiebt durch ein Sieb mit 1,5 mm-Maschenweite und mit Magnesiumstearat vermischt. Aus dieser Mischung werden Tabletten gepreßt.

Tablettengewicht: 400 mg
Durchmesser: 11 mm, rund, biplan, beidseitige Facette und einseitige Teilkerbe.

## Beispiel VIII

### Suppositorien, zu 200 mg 6-Äthoxy-benzothiazol-2(3H)-on

1 Zäpfchen enthält:

| | |
|---|---|
| Wirksubstanz | 0,20 g |
| Hartfett (z.B. Witepsol H 19 und Witepsol W 45) | 1,50 g |
| | 1,70 g |

### Herstellung:

Das Hartfett wird geschmolzen. Bei 38°C wird die gemahlene Wirksubstanz in der Schmelze homogen dispergiert. Es wird auf 35°C abgekühlt und in schwach vorgekühlte Suppositorienformen ausgegossen.
Zäpfchengewicht: 1,7 g.

## Beispiel IX

### Suspension mit 200 mg 6-Äthoxy-benzothiazol-2(3H)-on

100 ml Suspension enthalten:

| | |
|---|---|
| Wirksubstanz | 4,0 g |
| Carboxymethylcellulose | 0,1 g |
| p-Hydroxybenzoesäuremethylester | 0,05g |
| p-Hydroxybenzoesäurepropylester | 0,01g |
| Rohrzucker | 10,0 g |
| Glycerin | 5,0 g |

| | | |
|---|---|---|
| Sorbitlösung 70 % | | 20,0 g |
| Aroma | | 0,3 g |
| Wasser dest. | ad | 100,0 ml |

Herstellungsverfahren:

Dest. Wasser wird auf 70°C erhitzt. Hierin wird unter Rühren p-Hydroxybenzoesäuremethylester und -propylester sowie Glycerin und Carboxymethylcellulose gelöst. Es wird auf Raumtemperatur abgekühlt und unter Rühren die Wirksubstanz zugegeben und homogen dispergiert. Nach Zugabe und Lösen des Zuckers, der Sorbitlösung und des Aromas wird die Suspension zur Entlüftung unter Rühren evakuiert.

5 ml Suspension enthalten 200 mg Wirksubstanz.

## Patentansprüche

1.) Arzneimittel enthaltend ein oder mehrere Benzothiazol-2(3H)-one der allgemeinen Formel

,(I)

in der R ein Wasserstoffatom, die Methyl-, Methoxy- oder Ethoxygruppe bedeutet, neben üblichen Träger- und/oder Hilfsstoffen.

2.) Arzneimittel enthaltend ein oder mehrere in 6-Stellung substituierte Benzothiazol-2(3H)-one der allgemeinen Formel

,(I)

in der R die Methyl-, Methoxy- oder Ethoxygruppe bedeutet, neben üblichen Träger- und/oder Hilfsstoffen.

3.) Arzneimittel enthaltend 6-Ethoxy-benzothiazol-2(3H)-on neben den üblichen Träger- und/oder Hilfsstoffen.

4.) Arzneimittel enthaltend 6-Methoxy-benzothiazol-2(3H)-on neben den üblichen Träger- und/oder Hilfsstoffen.

5.) Arzneimittel enthaltend 6-Methyl-benzothiazol-2(3H)-on neben den üblichen Träger- und/oder Hilfsstoffen.

6.) Arzneimittel zur Bekämpfung von Schmerzzuständen, enthaltend Benzothiazol-2(3H)-on.

7.) Arzneimittel enthaltend eine Verbindung der allgemeinen Formel I, gemäß Anspruch 1, als einzigen Wirkstoff neben üblichen Träger- und/oder Hilfsstoffen.

8.) Die Verwendung der Verbindungen der allgemeinen Formel I gemäß Anspruch 1 bei der Bekämpfung von Schmerzzuständen.

9.) Die Verwendung von Verbindungen der allgemeinen Formel I gemäß Anspruch 1 zur Herstellung von Arzneimitteln auf nicht-chemischem Wege.

10.) Analgetika enthaltend eine oder mehrere Verbindungen der allgemeinen Formel I gemäß Anspruch 1 neben den üblichen Träger- und/oder Hilfsstoffen.

0022213

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung
EP 80 10 3571

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D,X | DIE PHARMAZIE, Band 19, Nr. 4, April 1964, Seiten 281-283 Berlin, DE. W. NIMMICH: "Über die antimikrobielle Wirksamkeit des Benzthiazolons und seiner Derivate" * Insgesamt * ---- | 1,6-10 |

KLASSIFIKATION DER ANMELDUNG (Int.Cl.³)
A 61 K 31/425 //
C 07 D 277/68

RECHERCHIERTE SACHGEBIETE (Int. Cl.³)
A 61 K 31/425
C 07 D 277/68

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-7,9,10
Unvollständig recherchierte Patentansprüche:
Nicht recherchierte Patentansprüche: Anspruch 8. Verfahren
Grund für die Beschränkung der Recherche: zur chirurgischen oder therapeutischen Behandlung des menschlichen oder tierischen Körpers. (Siehe Art. 52(4) des Europäischen Patentübereinkommens).

KATEGORIE DER GENANNTEN DOKUMENTE
X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| Den Haag | 12-09-1980 | HENRY |